# EUROPÄISCHE PATENTSCHRIFT

(11) **EP 0 493 719 B1**
(45) Veröffentlichungstag und Bekanntmachung des Hinweises auf die Patenterteilung: **06.09.1995**
(21) Anmeldenummer: 91121300.7
(22) Anmeldetag: 12.12.1991
(51) Int. Cl.: A61F 5/56

(54) **Vorrichtung zur Unterbindung des Schnarchens**
Device for snoring prevention
Dispositif pour empêcher le ronflement

(30) Priorität: 04.01.1991 DE 9100060 U
(43) Veröffentlichungstag der Anmeldung: 08.07.1992
(73) Patentinhaber: BRUCKHOFF APPARATEBAU GmbH, D-30159 Hannover (DE)
(72) Erfinder: Bruckhoff, Henning, W-3000 Hannover 1 (DE)
(74) Vertreter: Brümmerstedt, Hans Dietrich, Dipl.-Ing.

(56) Entgegenhaltungen:
- EP-A- 0 145 160
- WO-A-87/02577
- WO-A-87/05494
- US-A- 3 998 209

## Beschreibung

Die Erfindung betrifft eine Vorrichtung zur Unterbindung des Schnarchens einer schlafenden Person mit einem Mikrofon zur Aufnahme der Schnarchgeräusche und mit einer Auslösevorrichtung für bei Auftreten von Schnarchgeräuschen auf die Person einwirkenden Stimulationsmitteln.

Eine derartige Vorrichtung ist beispielsweise aus der WO-A-8 702 577 bekannt.

Bei bekannten Vorrichtungen dieser Art ist das Mikrofon im Raum aufgestellt oder am Bett befestigt, und die Stimulationsmittel sind akustischer oder mechanischer Art und bestehen beispielsweise aus Rüttelvorrichtungen für die Auflage oder das Kopfkissen des Schlafenden oder Geräuscherzeugern, die teilweise erst durch die schnarchende Person selbst wieder ausgeschaltet werden müssen und dann ein völliges Wachwerden erfordern. Sie haben ferner den Nachteil, daß dadurch im gleichen Raum schlafende Personen mehr als durch das Schnarchen selbst gestört werden können. Außerdem besteht die Gefahr, daß die Vorrichtung auch durch andere Geräusche als Schnarchen ausgelöst werden kann.

Der Erfindung liegt die Aufgabe zugrunde, eine Vorrichtung der eingangs genannten Art zu schaffen, die nur auf die schnarchende Person einwirkt und diese in ihrer gewohnten Schlafweise nicht beeinflußt, und die unempfindlich gegen andere Geräusche ist.

Die gestellte Aufgabe wird gemäß der Erfindung dadurch gelöst, daß das Mikrofon, die Auslösemittel und die Stimulationsmittel an der Innenseite einer am Kopf der Person befestigbaren Abdeckung für wenigstens ein Auge angeordnet sind, und daß die Stimulationsmittel aus einer mit vorgegebener Frequenz blinkenden und für eine vorgegebene Zeitdauer einschaltbaren Lichtquelle bestehen.

Neben dem Vorteil, daß andere im gleichen Raum schlafende Personen beim Wirksamwerden der Vorrichtung nicht gestört werden, besteht der Vorteil, daß die Vorrichtung unabhängig und überall einsatzfähig ist, z.B. in Bahn, Flugzeug und Freizeitunternehmungen.

Dabei bilden das Mikrofon, Die Auslösemittel und die Stimulationsmittel ein Modul, das lösbar an der Abdeckung befestigt ist, wobei vorzugsweise als Befestigung ein Klettverschluß dient. Die Abdeckung kann dann bei Beschädigung ersetzt oder bei Verschmutzung gereinigt werden.

Die Abdeckung ist vorzugsweise als Schlafbrille ausgebildet, so daß beide Augen bedeckt sind, wobei als Schlafbrille ein flexibles Gebilde, vorzugsweise aus Stoff, verstanden wird, das mit Bügeln oder dergl. an den Ohren oder mit einem Gummiband am Kopf festgelegt wird. Die Abdeckung kann aber auch als Augenklappe zum Umbinden und zur Abdeckung nur eines Auges ausgebildet. Dadurch wird die schlafende Person nicht in ihrer gewohnten Schlafweise beeinträchtigt.

In weiterer Ausgestaltung der Erfindung ist ein Schalter vorgesehen, mit dem die Helligkeit der Lichtquelle stufenweise einstellbar ist. Hierdurch ist eine individuelle Anpassung der Lichtverhältnisse gegeben, wobei durch Einstellung auf eine geringe Helligkeit Batteriestrom eingespart werden kann.

Es kann aber auch ein Schalter zur Einstellung der Ansprechschwelle der Auslösemittel für die Lichtquelle vorgesehen werden, so daß eine individuelle Berücksichtigung, bezogen auf das Schnarchgeräusch, möglich ist.

In vorteilhafter Ausgestaltung der Erfindung besteht die Lichtquelle aus wenigstens einer Leuchtdiode. Diese vermittelt trotz ihrer unmittelbaren Nähe vor dem geschlossenen Augenlid Lichtreize, die keinen Schreck verursachen, die aber ausreichen, daß der Schnarchende aus seinem Tiefschlaf in einen leichteren Schlaf geholt wird.

Es hat sich eine sehr gute Wirksamkeit der Vorrichtung gezeigt, wenn die Blinkfrequenz der Leuchtdiode kleiner als 4 Hz und vorzugsweise etwa 1 Hz beträgt.

Ferner ist es zweckmäßig, die Einschaltzeitdauer der Leuchtdiode auf nicht mehr als 6 Sekunden, vorzugsweise auf 3 Sekunden, einzustellen. Sie entspricht damit etwa der Atemfrequenz.

Dabei ist in weiterer Ausgestaltung der Erfindung vorgeshen, daß die Einschaltzeitdauer sich jeweils um die vorgegebene Dauer verlängert, wenn die Schnarchgeräusche über die vorgegebene Einschaltzeitdauer hinaus anhalten.

Ein ungewolltes Wirksamwerden der Vorrichtung durch andere Geräusche als Schnarchen ist praktisch ausgeschlossen, weil sich das Mikrofon in unmittelbarer Nähe der Schnarchquelle befindet.

Ferner ist es zweckmäßig, auf der Rückseite der Abdeckung einen Reflektor anzubringen, der beispielsweise aus einem hellen Stoff besteht. Hierdurch wird eine größere Streuung des von den Leuchtdioden abgegebenen Lichtes erreicht, so daß diese nicht unmittelbar auf die Augen gerichtet werden müssen.

Die Erfindung wird nachfolgend anhand der Zeichnung näher erläutert. In der Zeichnung zeigen:
- Fig. 1: eine schematische Darstellung eines Ausführungsbeispiels der erfindungsgemäßen Vorrichtung in Form einer Schlafbrille,
- Fig. 2: eine schematische Darstellung eines anderen Ausführungsbeispiels der erfindungsgemäßen Vorrichtung in Form einer Augenklappe und
- Fig. 3 und 4: Diagramme zur Veranschaulichung der Funktionsweise der erfindungsgemäßen Vorrichtung.

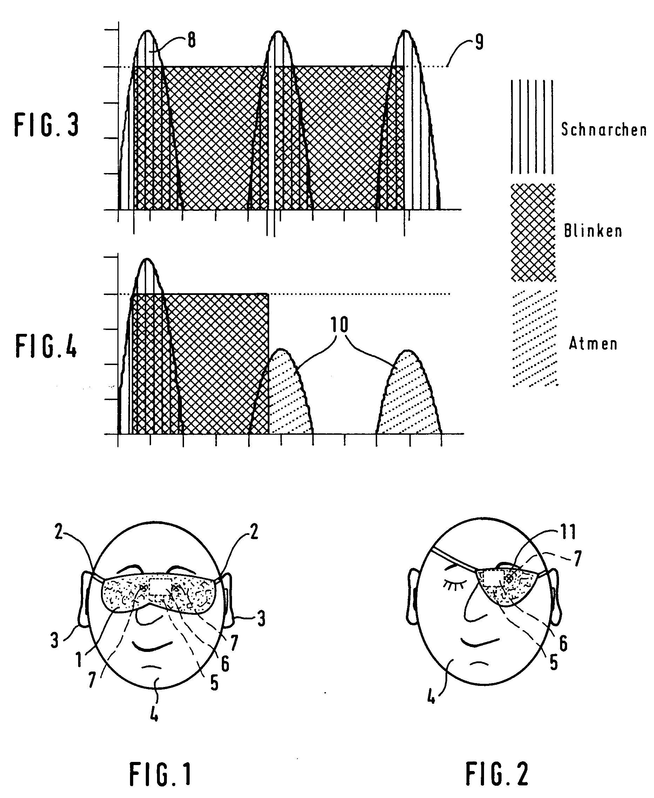

Bei dem in Fig. 1 dargestellten Ausführungsbeispiel ist die Vorrichtung als Brille 1 ausgebildet, die mittels eines oberhalb der Ohren 3 einer Person 4 zu deren Hinterkopf geführten Bandes 2 festgelegt werden kann, und wobei am Band 2 bekannte Einstellmittel zur Anpassung an die Kopfgröße vorgesehen sind. Die Brille 1 besteht aus weichem Material, z.B. aus dunklem Stoff, so daß sie im Gebrauch weder drückt noch anderweitig in der jeweiligen Schlafstellung des Kopfes stört. Auf der Innenseite der Brille 1 ist ein Mikrofon 5 so angeordnet, daß es möglichst nahe an Nase und Mund der Person 4 liegt, so daß es überwiegend die Schnarchgeräusche über Körperschall empfängt und dadurch andere Außengeräusche weitgehend ohne Einfluß bleiben. Außer dem Mikrofon 5 ist in der Brille ein an das Mikrofon angeschlossener Audioprozessor 6 als Auslösevorrichtung für zwei Leuchtdioden 7 vorgesehen. Das Mikrofon, der Audioprozessor und die Leuchtdioden 7 bilden einen einteiligen Modul, der lösbar an der Brille 1 angebracht ist, wobei als Befestigungsmittel vorzugsweise ein Klettverschluß dient. An dem Modul können - wie bei einer normalen Brille - weiche Seitenstege oder ein Sattelsteg zur Auflage am Nasenrücken angebracht sein. Der Audioprozessor bringt die Leuchtdioden 7 zum Blinken mit einer Frequenz, die kleiner als 4 Hz, vorzugsweise 1 Hz ist, wobei die Funktion der Leuchtdioden 7 eingeschaltet wird, sobald vom Mikrofon 5 Schnarchgeräusche 8 aufgefangen werden und diese gemäß Fig. 3 in ihrer Amplitude A eine Ansprechschwelle 9 überschreiten. Die Zeitdauer, während der die Leuchtdiode anspricht, wird auf weniger als 6 Sekunden - und vorzugsweise etwa 3 Sekunden - bemessen und entspricht somit etwa dem in Fig. 4 dargestellten Rhythmus der Atmung 10.

Auf der Innenseite der Schlafbrille 1 ist ein nicht dargestellter Reflektor angebracht, der vorzugsweise aus einem hellen Stoff besteht. Hierdurch wird das von den Leuchtdioden 7 abgegebene Licht gestreut, so daß die Leuchtdioden 7 nicht unmittelbar auf die Augen ausgerichtet zu werden brauchen.

Versuche haben gezeigt, daß nach längerem Gebrauch der erfindungsgemäßen Vorrichtung ein Trainingseffekt eintritt. Sobald der Benutzer das Lichtsignal registriert, weiß er unbewußt, daß er geschnarcht hat und begibt sich in eine andere Schlaflage.

Sollte das Schnarchen nach 3 Sekunden noch weiter anhalten, verlängert sich die Blinkdauer der Leuchtdioden 7 um weitere 3 Sekunden usw. Erst nach Einstellen des Schnarchens hören die Leuchtdioden 7 auf zu blinken. Mit einem nicht dargestellten mehrstufigen Schalter kann die Helligkeit der Leuchtdioden stufenweise eingestellt werden. Es sollte dabei immer die kleinstmögliche Helligkeit gewählt werden, um Batteriestrom zu sparen. Es kann aber auch ein Schalter vorgesehen werden, mit dem die Ansprechschwelle 9 und damit die Empfindlichkeit der Vorrichtung eingestellt werden kann.

Die in Fig. 2 dargestellte Ausführungsform unterscheidet sich von Fig. 1 nicht in ihrer Funktion, sondern nur in ihrer Ausbildung als Augenklappe 11 die um den Kopf umgebunden wird und nur ein Auge bedeckt, was als Vorteil empfunden werden kann, weil dann im noch wachen Zustand Wahrnehmungen im Raum möglich sind, z.B. die Ablesung der Uhrzeit.

## Patentansprüche

1. Vorrichtung zur Unterbindung des Schnarchens einer schlafenden Person mit einem Mikrofon zur Aufnahme der Schnarchgeräusche und mit einer Auslösevorrichtung für bei Auftreten von Schnarchgeräuschen auf die Person einwirkenden Stimulationsmitteln, dadurch gekennzeichnet, daß das Mikrofon (5), die Auslösemittel (6) und die Stimulationsmittel (7) an der Innenseite einer am Kopf (4) der Person befestigbaren Abdeckung (1, 11) für wenigstens ein Auge angeordnet sind, und daß die Stimulationsmittel aus einer mit vorgegebener Frequenz blinkenden und für eine vorgegebene Zeitdauer einschaltbaren Lichtquelle (7) bestehen.

2. Vorrichtung nach Anspruch 1, dadurch gekennzeichnet, daß das Mikrofon (5), die Auslösemittel (6) und die Stimulationsmittel (7) ein Modul bilden, das lösbar an der Abdeckung befestigt ist.

3. Vorrichtung nach Anspruch 2, dadurch gekennzeichnet, daß als Befestigung ein Klettverschluß dient.

4. Vorrichtung nach Anspruch 1 bis 3, dadurch gekennzeichnet, daß die Abdeckung eine Schlafbrille (1) ist.

5. Vorrichtung nach Anspruch 1 bis 3, dadurch gekennzeichnet, daß die Abdeckung eine Augenklappe (11) ist.

6. Vorrichtung nach einem der vorhergehenden Ansprüche, dadurch gekennzeichnet, daß ein Schalter zur stufenweisen Einstellung der Helligkeit der Lichtquelle vorgesehen ist.

7. Vorrichtung nach einem der vorhergehenden Ansprüche, dadurch gekennzeichnet, daß ein Schalter zur Einstellung der Ansprechschwelle (9) der Auslösemittel (6) für die Leuchtdioden (7) vorgesehen ist.

8. Vorrichtung nach einem der vorhergehenden Ansprüche, dadurch gekennzeichnet, daß die Lichtquelle (7) aus wenigstens einer Leuchtdiode besteht.

9. Vorrichtung nach Anspruch 8, dadurch gekennzeichnet, daß jedem Auge eine Leuchtdiode (7) zugeordnet ist.

10. Vorrichtung nach Anspruch 8 oder 9, dadurch gekennzeichnet, daß die Leuchtdioden-Blinkfrequenz kleiner als 4 Hz ist.

11. Vorrichtung nach Anspruch 10, dadurch gekennzeichnet, daß die Leuchtdioden-Blinkfrequenz 1 Hz ist.

12. Vorrichtung nach einem der vorhergehenden Ansprüche, dadurch gekennzeichnet, daß die Leuchtdioden-Einschaltzeitdauer nicht mehr als 6 Sekunden beträgt.

13. Vorrichtung nach Anspruch 12, dadurch gekennzeichnet, daß die Leuchtdioden-Einschaltzeitdauer 3 Sekunden beträgt.

14. Vorrichtung nach Anspruch 12 oder 13, dadurch gekennzeichnet, daß die Einschaltzeitdauer sich jeweils um die vorgegebene Dauer verlängert, wenn die Schnarchgeräusche über die vorgegebene Einschaltzeitdauer hinaus anhalten.

15. Vorrichtung nach einem der vorhergehenden Ansprüche, dadurch gekennzeichnet, daß auf der Rückseite der Abdeckung ein Reflektor angebracht ist.

16. Vorrichtung nach Anspruch 15, dadurch gekennzeichnet, daß der Reflektor aus einem hellen Stoff besteht.

## Claims

1. Device for preventing the snoring of a sleeping person, comprising a microphone for receiving the snoring noise and comprising a triggering device for stimulation means acting upon the person when snoring noise occurs, characterized in that the microphone (5), the triggering means (6) and the stimulation means (7) are disposed on the inner face of a cover (1, 11) for at least one eye, which can be secured to the head (4) of the person, and that the stimulation means consist of a light source (7) blinking with a predetermined frequency and able to be switched on for a predetermined period.

2. Device according to Claim 1, characterized in that the microphone (5), the triggering means (6) and the stimulation means (7) form a module, which is releasably secured to the cover.

3. Device according to Claim 2, characterized in that a burr closure serves as the fixing.

4. Device according to Claims 1 to 3, characterized in that the cover is a sleeping goggles (1).

5. Device according to Claims 1 to 3, characterized in that the cover is an eye-patch (11).

6. Device according to one of the preceding Claims, characterized in that a switch for the incremental adjustment of the brightness of the light source is provided.

7. Device according to one of the preceding Claims, characterized in that a switch for the adjustment of the response threshold (9) of the triggering means (6) for the light emitting diodes (7) is provided.

8. Device according to one of the preceding Claims, characterized in that the light source (7) consists of at least one light emitting diode.

9. Device according to Claim 8, characterized in that a light emitting diode (7) is associated with each eye.

10. Device according to Claim 8 or 9, characterized in that the blinking frequency of the light emitting diodes is less than 4 Hz.

11. Device according to Claim 10, characterized in that the blinking frequency of the light emitting diodes is 1 Hz.

12. Device according to one of the preceding Claims, characterized in that the switched-on time of the light emitting diodes is not greater than 6 seconds.

13. Device according to Claim 12, characterized in that the switched-on time of the light emitting diodes is 3 seconds.

14. Device according to Claim 12 or 13, characterized in that the switched-on time is increased on each occasion by the predetermined time when the snoring noise continues beyond the predetermined switched-on time.

15. Device according to one of the preceding Claims, characterized in that a reflector is mounted on the rear face of the cover.

16. Device according to Claim 15, characterized in that the reflector is of a bright material.

## Revendications

1. Dispositif pour empêcher le ronflement d'une personne endormie comportant un microphone pour la réception du bruit de ronflement et un dispositif de déclenchement de moyens de stimulation agissant sur la personne lors de l'apparition du bruit, caractérisé en ce que le microphone (5), les moyens de déclenchement (6) et les moyens de stimulation (7) sont disposés sur le côté interne d'un couvre-oeil (1, 11) qui peut être fixé sur la tête (4) de la personne, en ce qui concerne au moins un oeil, et en ce que les moyens de stimulation sont constitués par une source lumineuse (7) clignotant à une fréquence prédéterminée et qui peut être mis en marche pendant un temps prédétermine.

2. Dispositif suivant la revendication 1, caractérisé en ce querophone (5), les moyens de déclenchement (6) et les moyens de stimulation (7) forment un module qui est est monté amovible sur le couvre-oeil.

3. Dispositif suivant la revendication 2, caractérisé en ce que, comme fixation, on utilise une bande agrippante.

4. Dispositif suivant l'une des revendications 1 à 3, caractérisé en ce que le couvre-oeil est une paire de lunettes de sommeil (1).

5. Dispositif suivant l'une des revendications 1 à 3, caractérisé en ce que le couvre-oeil (11) ne recouvre qu'un oeil.

6. Dispositif suivant l'une des revendications précédentes, caractérisé en ce qu'un circuit est prévu pour le réglage échelonné de la luminosité de la source lumineuse.

7. Dispositif suivant l'une des revendications précédentes, caractérisé en ce qu'un circuit est prévu pour le réglage du seuil de réponse (9) des moyens de déclenchement (6) de diodes lumineuses.

8. Dispositif suivant l'une des revendications précédentes, caractérisé en ce que la source lumineuse (7) consiste en au moins une diode lumineuse.

9. Dispositif suivant la revendication 8, caractérisé en ce qu'à chaque oeil est attribuée une diode lumineuse (7).

10. Dispositif suivant la revendication 8 ou 9, caractérisé en ce que la fréquence de clignotement des diodes lumineuses est inférieure à 4 Hz.

11. Dispositif suivant la revendication 10, caractérisé en ce que la fréquence de clignotement des diodes lumineuses est de 1 Hz.

12. Dispositif suivant l'une des revendications précédentes, caractérisé en ce que la durée de marche des diodes lumineuses ne dure pas plus de 6 secondes.

13. Dispositif suivant la revendication 12, caractérisé en ce que la durée de marche des diodes lumineuses dure 3 secondes.

14. Dispositif suivant la revendication 12 ou 13, caractérisé en ce que la durée de marche se prolonge chaque fois passé la durée prédéterminée si les bruits de ronflement s'arrêtent au-delà de la durée de marche prédéterminée.

15. Dispositif suivant l'une des revendications précédentes, caractérisé en ce que, sur le côté arrière du couvre-oeil, il y a un réflecteur.

16. Dispositif suivant la revendication 15, caractérisé en ce que le réflecteur consiste en une matière claire.
